# EUROPEAN PATENT SPECIFICATION

(11) **EP 2 377 059 B1**
(45) Date of publication and mention of the grant of the patent: **08.01.2020**
(21) Application number: 09802007.6
(22) Date of filing: 19.11.2009
(51) Int. Cl.: G16H 10/60, G06Q 50/00

(54) **AN ASSERTION-BASED RECORD LINKAGE IN DISTRIBUTED AND AUTONOMOUS HEALTHCARE ENVIRONMENTS**
AUF BEHAUPTUNG BASIERENDE AUFZEICHNUNGSVERKNÜPFUNG IN VERTEILTEN UND AUTONOMEN GESUNDHEITSVERSORGUNGSUMGEBUNGEN
ASSOCIATION DE FICHIERS FONDÉE SUR DES AFFIRMATIONS DANS DES ENVIRONNEMENTS MÉDICAUX AUTONOMES ET RÉPARTIS

(30) Priority: 12.12.2008 US 121989 P
(43) Date of publication of application: 19.10.2011
(73) Proprietor: Koninklijke Philips N.V., 5656 AE Eindhoven (NL)
(72) Inventor: VDOVJAK, Richard, NL-5621 BA Eindhoven (NL); BUCUR, Anca, Ioana, Daniela, NL-5621 BA Eindhoven (NL)
(74) Representative: van Velzen, Maaike Mathilde
(86) International application number: PCT/IB2009/055185
(87) International publication number: WO 2010/067230

(56) References cited:
- WO-A2-01/22285
- WO-A2-03/021485
- US-A1- 2006 287 890
- GARCIA M S ET AL: "Immunization RegistRIES DeDuplication and Record Matching" INTERNET CITATION 1999, XP002259647 Retrieved from the Internet: URL:www.immunizationregistries.com/white_p apers/ WHP006A_deduplication_recordmatching.pdf -> [retrieved on 2003-10-27]
- "THE FOUR CHALLENGES OF CUSTOMER-CENTRIC DATA WAREHOUSING" INTERNET CITATION 1998, XP002259648 Retrieved from the Internet: URL:http://www.ctiforum.com/technology/CRM /whitepaper/dwo.pdf> [retrieved on 2003-10-27]

## Description

The present application relates to the art of data continuity. It finds particular application to identifying individual patients and patient medical records in order to communicate and share medical information among different healthcare facilities and will be described with particular reference thereto. However, it will also find use in other types of data display applications in which data continuity is of interest.

Patients commonly receive medical care from multiple healthcare providers, many of which are geographically dispersed and located at multiple sites. Using multiple healthcare providers results in an individual patient receiving multiple patient identifiers, each patient identifier local to a specific healthcare provider. Patient data such as medical tests, histories, doctors' reports, medical images and other relevant medical information is spread across multiple healthcare provider sites. In order for a healthcare provider to retrieve patient data records stored among multiple healthcare provider sites, it is necessary to reconcile the multiple patient identifiers of the corresponding healthcare providers and to link the multiple patient identifiers together.

Patients do not always give their name consistently, e.g., with or without a middle initial, diminutive or full first name, with or without a name suffix such as Jr., married or maiden name, etc. Not only are addresses sometimes given inconsistently, but people also move. Patients in the same family may have similar names, similar addresses, and also similar medical information.

WO 01/22285 describes a method of training a system from examples achieves high accuracy by finding the optimal weighting of different clues indicating whether two data items such as database records should be matched or linked. The trained system provides three possible outputs when presented with two data items: yes, no or I don't know (human intervention required). A maximum entropy model can be used to determine whether the two records should be linked or matched. Using the trained maximum entropy model, a high probability indicates that the pair should be linked, a low probability indicates that the pair should not be linked, and intermediate probabilities are generally held for human review.

WO 03/021485 describes a system preventing the creation of duplicate records and identifies, groups, and consolidates duplicate records and manages the associated workload. A method consolidates multiple records that are associated with a single entity and are stored in at least one record repository. The method involves identifying first and second records and applying record matching criteria to compare data element content of the first and second identified records to determine commonality data. The commonality data is indicative of a likelihood the first and second records are associated with a common entity. The first and second record content is merged into a composite record in response to the determined commonality data. One of the first and second records are selected as a surviving record based on earliest date of record creation or relative content of the first and second records.

There is currently a need for a system, a method, and a device that enables medical records to follow a patient as they travel between multiple healthcare providers.

The present application provides an improved method, system and apparatus which overcomes the above-referenced problems and others.

The invention is defined by the independent claims. The dependent claims define advantageous embodiments.

In accordance with one aspect, a method is proposed of reconciling customer records, which comprises assigning a unique record number to a customer record, then retrieving demographic information for a customer record to match the demographic information against demographic information in a collection of records in other systems. This is used to find records that belong to the same customer, and then compare the customer record demographic information with at least one other record demographic information in the collection of records in at least one other system to derive a likelihood ratio for each compared record, then compares each likelihood ratio to a defined accept threshold and to a defined reject threshold and finally attaches an assertion for the compared customer records based on at least one likelihood ratio comparison.

In accordance with another aspect, an apparatus is proposed for reconciling customer records which comprises input means for receiving an input customer record and accompanying demographic data, which uses a collection of customer records and accompanying demographic data. Also, a processing means is included for deriving a unique customer record number from the demographic data using a computational means for comparing at least one customer record demographic data of at least one customer record with other record demographic data in a collection of records in order to derive a likelihood ratio for each compared record. The computational then means compares each likelihood ratio to a defined accept threshold and to a defined reject threshold and performs one of either rejecting the at least one customer record if the likelihood ratio falls below a reject threshold; or accepting the at least one customer record if the likelihood ratio falls above an accept threshold; or identifying the at least one customer record for a manual review if the likelihood ratio falls between the accept threshold and the reject threshold. The means also records to a data storage medium whether the at least one customer record was rejected, accepted, or identified for manual review and recording whether an assertion is made by an institution concerning a pair of the customer records.

In accordance with another aspect, a method is proposed for reconciling medical patient records which comprises inputting a patient record, retrieving a plurality of patient records from a collection of stored patient records, compares the input patient record with the retrieved patient records, deriving a likelihood ratio from each pair of compared records, assigning a reject assertion in response to the likelihood ratio falling below a reject threshold level, assigning an accept assertion in response to the likelihood ratio falling above an accept threshold level, and finally placing the record on an exception list if the likelihood ratio falls between the accept threshold and the reject threshold.

An advantage resides in creating an index based on demographic data which will be the same or similar regardless of the evaluation procedures of the healthcare provider.

A further advantage is the maximization of the use of assertions in a manual review phase by allowing sites to see the assertions issued by other sites and to take them into account when desired.

Still further advantages of the present application will be appreciated to those of ordinary skill in the art upon reading and understand the following detailed description.

The present application may take form in various components and arrangements of components, and in various steps and arrangements of steps. The drawings are only for purposes of illustrating the preferred embodiments and are not to be construed as limiting the present application.
FIGURE 1 illustrates two thresholds for distinguishing three outcomes with respect to record matching.
FIGURE 2 illustrates assertion acceptance in distributed heterogeneous environment with autonomous sites.
FIGURE 3 illustrates examples of an assertion handling system for a federation containing four participating institutions.
FIGURE 4 presents a flow chart which illustrates the steps in the method claims.
FIGURE 5 presents an illustration of the information flow through the apparatus of an embodiment of the present application.

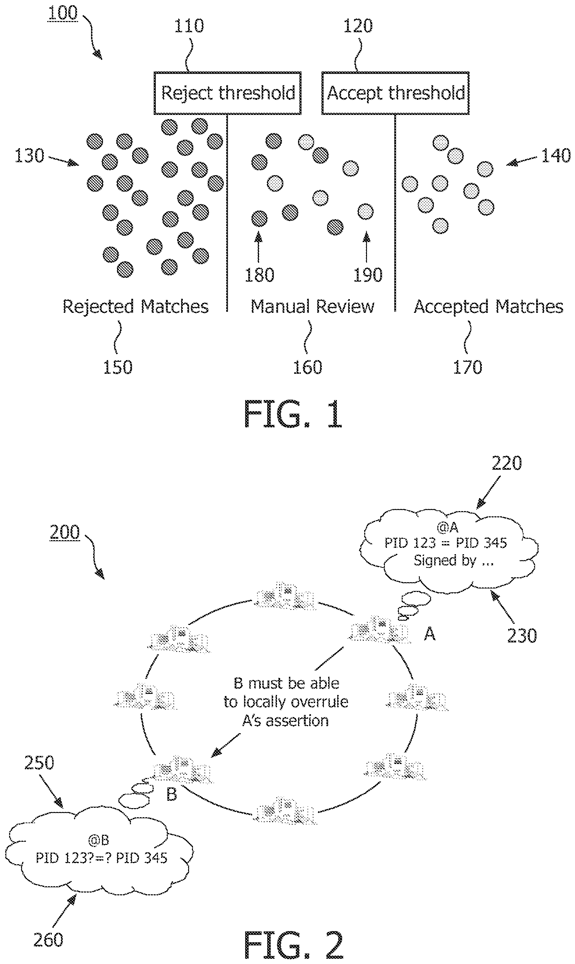

With reference to FIGURE 1, a probabilistic algorithm, which compares a fixed record with a number of candidates for a match, computes for each candidate a likelihood ratio or weighted score that is individually compared with reject 110 or accept 120 thresholds. The reject threshold 110 is used as a basis for a comparison used to decide whether the record falls below the reject threshold 110. If the likelihood ratio of a record is less than the reject threshold 110 the record is rejected 130 as not being a match 150 and the record is not linked. The accept threshold 120 is used as a basis for a comparison used to decide which records exceed the accept threshold. If the likelihood ratio of a record is greater than the accept threshold 120, then the record 140 is accepted as a match 170 and the record is linked. If the record is greater than the reject threshold 110 and also less than the accept threshold 120, then the computed likelihood falls between the accept and reject thresholds. Here, the record could be either rejected 180 or accepted 190. The decision is not made automatically, and is flagged for a manual review 160 by qualified personnel. The manual review is made to determine if the record should be properly rejected 180 or accepted 190 as a match. The manual review 160 of uncertain matches is of crucial importance in order to minimize linkage errors as those can have far-reaching consequences ultimately endangering patient health.

As medical providers or health systems associate or consolidate, it becomes advantageous to share patient records. As such, this may result in multiple provider databases containing medical record numbers (MRN) for the same patient. For each healthcare provider, the flow of information into and out of the patient record is channeled through a master patient index (MPI) that associates a unique medical record number (MRN) to each patient entity when a unique record exists. To obtain an enterprise-wide view on patients across distributed data sources, an enterprise master patient index (EMPI) is put in place. The EMPI is developed through integration of the individual MPIs of the sources. Generally, this integration of patient records is achieved by comparing demographic attributes such as first/last name, gender, date of birth, address, and other demographic data to create the EMPI as a form of an enterprise level patient identifier which may enable the same patient to be recognized in records compiled at different medical facilities by different medical providers. Such an enterprise level patient identifier is rarely based on a single identifier shared across the different organizations in the enterprise.

Probabilistic algorithms can be used to compare a fixed record with a number of candidates for a match, and to compute for each candidate a likelihood ratioor weighted score, that is compared to the chosen accept and reject thresholds, as explained above in connection with Fig. 1. This method is used to determine the probability that two different records at two separate medical facilities represent the same patient, and to decide whether to link the records or not to link the records. When a decision cannot be taken automatically, such as when the computed likelihood falls between the two thresholds, qualified personnel manually review or flag the potential matches before they are accepted, and also review the potential mismatches before they are rejected. The manual review of uncertain matches is very important in order to minimize linkage errors, but at the same time it is time-consuming and hence expensive.

For each medical facility, the flow of information into and out of the patient record is channeled through a master patient index (MPI) that associates a unique medical record number (MRN) to each patient entity when a unit record exists. To obtain an enterprise-wide view on patients across distributed data sources, an enterprise master patient index (EMPI) is put in place. The EMPI is developed through integration of the individual MPIs of the sources.

Currently, if two records are manually linked or manually declared as different, this fact is used as the "single ground truth" in the whole system. The problem with this approach is that the manual matching phase accepts as true a single authoritative decision. This decision may not be acceptable in other autonomous environments, where there is no enterprise wide authority recognized by all the sites and therefore no single source of truth. An enterprise-wide standard may be achieved through use of an assertion.

An assertion is attached to a pair of records to be matched, based on the likelihood ratio comparison, stating whether it is believed that the two records belong to the same patient or not. An assertion-based record linkage enables all participating sites to independently decide whether the relevant records submitted for manual review, in a federation of healthcare providers, belong to the same patient. None of the review decisions is taken as a single global ground truth. Individual assertions are maintained for every institution, serving as a local ground truth with respect to the institution that issued them, but not necessarily for other institutions.

With reference to FIGURE 2, a system 200 is shown by which two separate hospitals A and B share records and manually link two patient records together, as belonging to the same patient. Hospital A for example determines 220 that the two separate records e.g., identified by PID=123 and PID=345 respectively (where PID stands for patient identifier) are a match and makes an assertion 230 that the records are a match. Another institution hospital B is able to perform 250 a separate manual or automated review of its own, and make an assertion 260 that the records are not a match, thereby locally overruling the assertion 230 made at hospital A.

The ability of hospital B to overturn an assertion made by hospital A is an advantage of the present application. If hospital B, 240, were not able to overturn the hospital A assertion, then hospital B would lose autonomy over its own data and in principle could not guarantee data consistency. For instance, a mistake made at hospital A during the review, would, if hospital B were not able to overrule the assertion made by hospital A, force hospital B to become responsible for hospital A's error without being able to influence the matching outcome. However, as an autonomous organization, hospital B does not need to consider and apply decisions taken at hospital A.

In some current record linkage solutions, if two records are manually linked or manually declared as different, this fact is used as the "single ground truth" throughout the whole system. For example, record matching applied within a uniform enterprise-wide setting where the distributed sites with their own identification schemes become part of one larger "virtual" enterprise, e.g., by acquisitions or mergers. The "single ground truth" approach to manual review works well in such settings, because the degree of trust established among the participating parties is usually quite high and hence the results of the manual review are accepted by all parties without a doubt.

The model of complete trust that is essentially assumed in the "single ground truth" approach does not apply to all environments in which patient data is to be shared. Particularly, in environments where participating institutions are only loosely coupled and remain autonomous in their governance, the solution with only one single ground truth with respect to manual match review can cause problems. Some of the emerging RHIOs (Regional Health Information Organization) represent such distributed autonomous environments. There, participating institutions retain the complete control over their data and the quality process that is associated with handling it

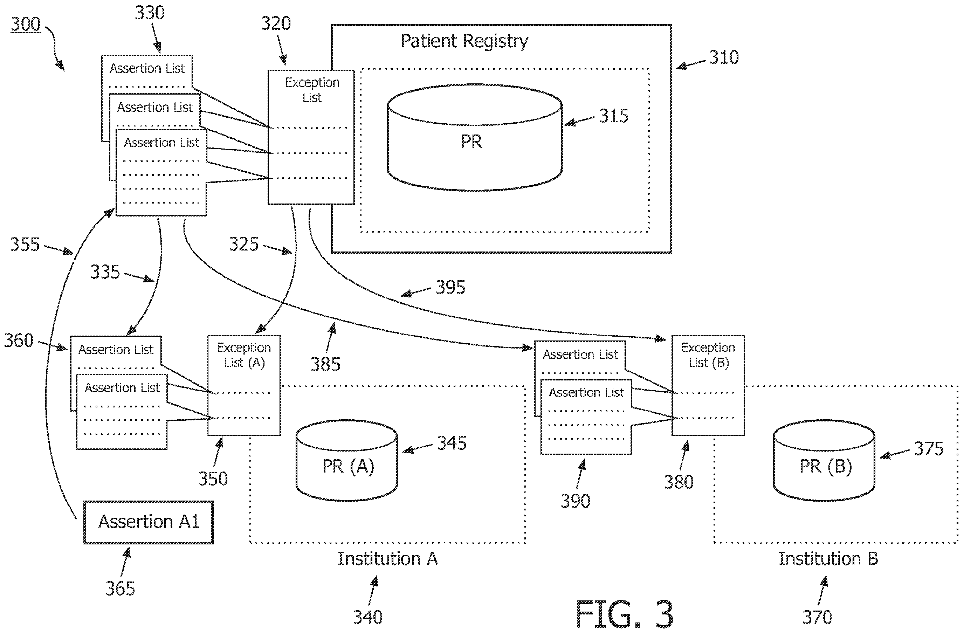

With reference to FIGURE 3, an assertion handling system 300 is shown in a scenario where are autonomous and linkage decisions are not made at the enterprise-wide level where the enterprise-wide level or federation level refers to two or more medical facilities using the present application. A enterprise-wide system that handles an enterprise-wide patient registry (PR) 310 and stores the data in a global database 315, builds a enterprise-wide exception list 320 with potential matches for manual review. Each entry in the global exception list 320 contains a plurality of local patient identifiers referencing a plurality of records that potentially match, wherein the contents of the records are used to determine a match and the identifiers are used to reference specific records used in the matching assertion determination process. The system 300 also includes an assertion list 330 which contains a list of assertions of matching or non-matching records represented by patient identifiers, and each entry in this assertion list 330 identifies the medical facility that made the assertion. The system also identifies additional information such as the user who made the assertion, and the timestamp when the assertion was made. The potential record matches of the enterprise-wide exception list 320 are compared to the assertions of matches or non-matches in the assertion list 330 to identify the same potentially matched records in both the assertion list 330 and the enterprise-wide exception list 320. Together with the corresponding part of the enterprise-wide exception list 320, the assertion list 330 is provided to the authorities of the local sites 335, 385, when the exceptions are being reviewed. The enterprise-wide exception list 320, together with already existing assertion list 330, is distributed 325, 395, to the participating institutions 340, 370 taking into account locally relevant information such as the split/distribution of the assertion list 330 which is determined by locally known patient identifiers. This local information is stored in a local patient registry or database 345, 375. Each site then proceeds with resolving its local exception list 350, 380 by making assertions 365 about matches or mismatches. These assertions 365 then propagate back 355 to the enterprise-wide exception list 320, where the assertions indicate that for that particular institution 340, 370, the provided assertion 365 constitutes a local ground truth. Submitted assertions 365 are also broadcasted, or added to the local exception lists 350, 380 of those medical facilities whose exception list 350, 380 contains the patient record about which the assertion 365 was made. When the assertion lists 360, 390, are locally resolved by each site 340, 370, the records in the local exception list 350, 380 already asserted by a different site are considered matches or mismatches for that site from that moment on. Those records will not be sent again to that site with the new local exception list. As an additional service, the system notifies the sites when the two conflicting assertions are made about one patient which may indicate a mistake during one of the review processes.

A medical facility reviews its own local exception list and also the corresponding assertion lists when the patient identifiers at the different healthcare organizations which are participating in the system are linked together.

Exceptions also need to be reviewed by an medical facility during normal system operation, each time an entry relevant for that site is added to the global exception list. Items are added to the global exception list during the system operation when a new patient is registered and the identity matching algorithm generates an exception for a possible match, in which case the exception list needs to be reviewed regularly.

The assertion list containing assertions already made at other medical facilities regarding the records to be evaluated may help the local site to decide whether the records should be linked, but it is not a source of truth. The local site makes its own assertions which are sent back to the patient registry 310 and stored in the global assertion list 330 as the truth for that site.

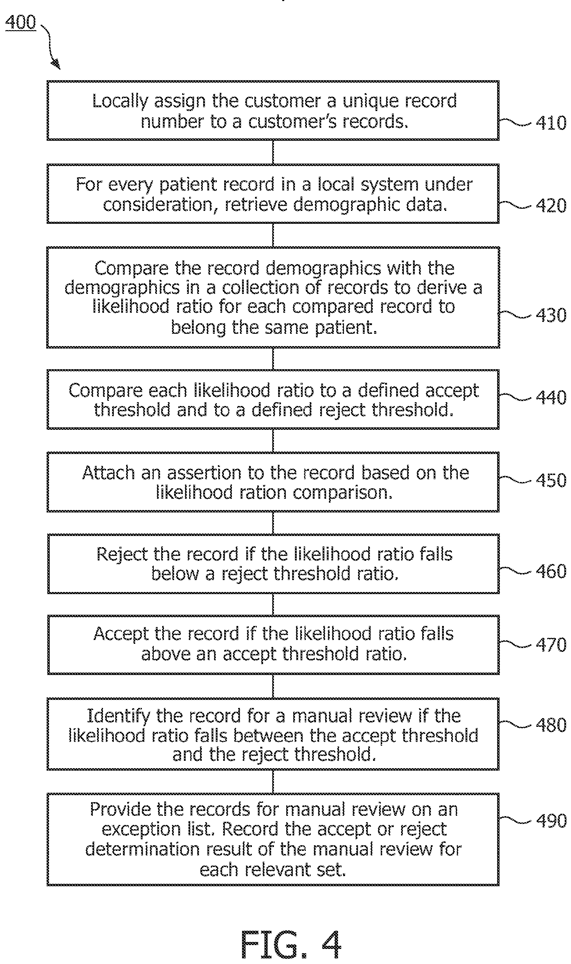

With reference to FIGURE 4, a series of steps of a method 400 for performing the present application are presented. A step or means 410 assigns a unique record number to a customer's record. Then, a step or means 420 retrieves the demographic data for a particular customer record in a system under consideration, to match that particular customer record against the demographic data in other systems in a federation to find records that belong to the same patient. Next, a step or means 430 compares the customer record demographic data with the demographic data in a collection of records to derive a likelihood ratio for each compared record. Next, a step or means 440 compares each likelihood ratio to a defined accept threshold and to a defined reject threshold. Then a step or means 450 attaches an assertion to the record based on the likelihood ratio comparison. Next, a step or means 460 rejects the record if the likelihood ratio falls below a reject threshold ratio. Then a step or means 470 accepts the record if the likelihood ratio falls above an accept threshold ratio. Then, a step or means 450 sets the record for a manual review if the likelihood ratio falls between the accept threshold and the reject threshold. Then, a step or means 490 places the records to be manually reviewed on an exception list and distributes this list to the relevant institutions, and records the determination of an accept or reject result made by manual review at each relevant institution.

The problem of patient identity in a federated environment in the absence of a global common identifier is a key issue, wherein the solving of such a key issue is considered to be a prerequisite to being able to build and deploy a Federated Picture Archiving and Communication System (PACS) solution. The present application addresses the manual review phase of the matching process in the context of autonomous environments.

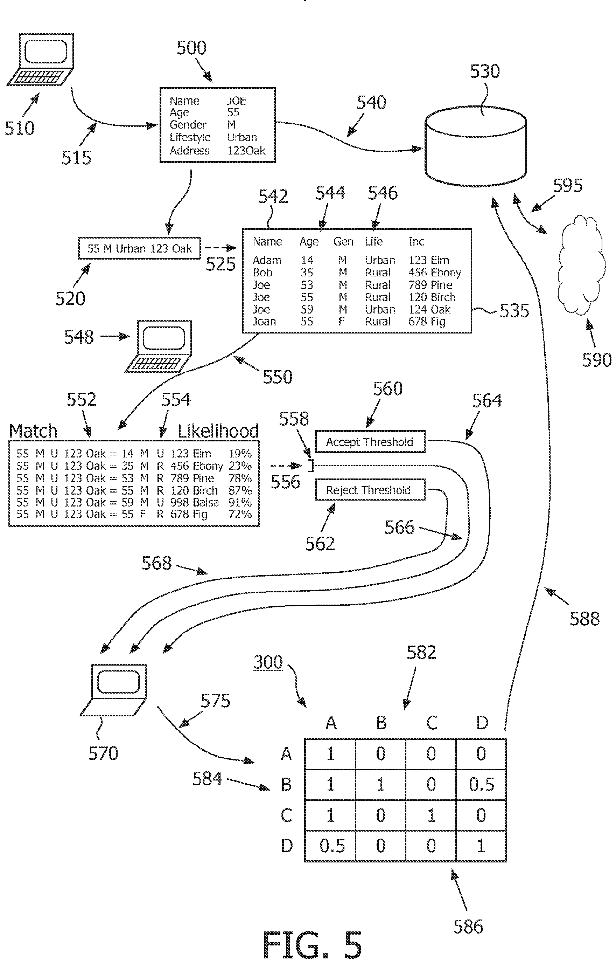

With reference to FIGURE 5, a description of the interaction of the data with the apparatus within the computer operable medium is described 500. Using input means 510, a record is input 515 using an input device such as, but not limited to, a computer terminal 510. The entered records reside in any type or a predetermined format 520. The record will also contain demographic data such as, but not limited to age, gender, race, urban or rural lifestyle, address, telephone number, and the like. The entered record demographic data is transmitted 535 to a database 530. The demographic data 540 will be used as a pointer 525. This pointer 525 will facilitate the search 552 of a collection of previously entered records 550 retrieved 535 from the database 530. The search will proceed, one record at a time, either sequentially as entered or in an order such as, but not limited to, alphabetically. Such a search will attempt to find a match between demographic data in the just entered new record 520 and demographic data of the records 550 in the existing database 530 based on how closely the demographic data in the new entered record 520 matches the demographic data of the individual records 550 in the database 530. Such a search is performed using a software means provided on a computer operable medium 560 and executable by a processor. From this matching 562, a likelihood ratio 564 is derived. The likelihood ratio 564 is compared 566 against a defined accept threshold 570 and against a defined reject threshold 572, producing one of the following three results. If the likelihood ratio 564 is greater than or equal to the accept threshold 570, then the value is accepted as a match and a positive assertion is made that this is a match 576. If the likelihood ratio 564 is less than or equal to the reject value 572, then the value is rejected as not being a match and a negative assertion is made that this is not a match 578. If the ratio is less than the accept threshold 570 and also more than the reject threshold 572, then the record is flagged 574 and the record will be placed on an exception list. Records on this exception list will be submitted for a manual review. Such a manual review may comprise a determination being made as to whether a match between two records placed on the exception list should be accepted or rejected, and attaching an assertion to the pair of records. This assertion should be entered manually into a computer 580. The exception list may also be placed in a computer. Each site holding one of the records independently asserts whether the two records belong to the same patient or not. This determination becomes the grounds of the assertion. Once such a determination is made, an assertion of accept or rejection is made, and this assertion 585 is stored in the system database 530. The assertions recorded in the database may be disseminated to other users 595 by porting the database 530 of assertions onto a network 590.

For example, the entered record in the present example is for Joe, a 55 year old male urban dweller. A comparison with a record in the database of Adam, a 14 year old male urban dweller would produce a low 19% likelihood ratio of a match due to the great age and address disparity between the two compared records. If the threshold ratio of rejection were 20%, then this record with a 19% ratio would fall below the 20% rejection threshold and would be rejected. These two compared records are probably not for the same person.

Another comparison, this time of the entered record of Joe the 55 year old male urban dweller with the database record of another different Joe who is 59 years old, a male urban dweller would produce a higher likelihood ratio of 91% because these two records are a much closer match in terms of age, gender, and lifestyle. If the threshold ratio for accept were 90%, then this record with an acceptance ratio of 91% would be above the 90% acceptance ratio and would be accepted as a match. These two compared records are likely for the same person.

A comparison with a record for Joan, a 55 year old female rural dweller would produce a likelihood ratio of 72%. As this 72% is above the 20% reject ratio and also below the 90% accept ratio, this record would be placed on an exception list and flagged for manual review. Only a manual review could determine whether these records are for the same person.

Each demographic factor can be, but is not necessarily, equally weighted. For example, an individual's address can change a great deal in a short period of time. Therefore this demographic might be weighted to be of less importance than other more stable, less inclined to change demographics. A demographic that rarely or never changes, such as gender or race, might be given greater weight because this demographic may be more reliable as an indicator of a specific person. In the present example, this would explain why the likelihood match between Joe 55 M U 123 Oak and Joe 59 M U 998 Balsa is at 91% despite the difference in address between these two individuals. Here, the similarity in age, gender, and lifestyle is weighted more heavily than address is weighted.

The above-described process is performed on one or more computers or computer systems. Computer programs for performing the steps can be stored on a tangible computer readable medium, such as a disc, computer memory, or the like.

A plurality of healthcare providers can exchange information and review each other's evaluations of patient medical records when determining the likelihood ratio that two records submitted for manual review belong to the same patient.

The present application has been described with reference to the preferred embodiments. Modifications and alterations may occur to others upon reading and understanding the preceding detailed description. It is intended that the present application be constructed as including all such modifications and alterations insofar as they come within the scope of the appended claims or the equivalents thereof.

## Claims

1. A computer implemented method of reconciling customer records between a plurality of sites, comprising:
assigning a unique record number to a customer record (410);
retrieving demographic information for a customer record in a system under consideration, to match said demographic information against demographic information in a collection of records in other systems to find records that belong to the same customer (420);
comparing the customer record demographic information with at least one other record demographic information in the collection of records in at least one other system to derive a likelihood ratio for each compared record (430);
comparing each likelihood ratio to a defined accept threshold and to a defined reject threshold (440), wherein the likelihood ratio comparison comprises one of:
- rejecting the compared customer records if the likelihood ratio falls below a reject threshold ratio (460),
- accepting the compared customer records if the likelihood ratio falls above an accept threshold ratio (470), and
- identifying the customer records for a manual review if the likelihood ratio falls between the accept threshold and the reject threshold (480); the method further comprising:
attaching an assertion for the compared customer records based on at least one likelihood ratio comparison (450); and
placing the customer records to be manually reviewed on an exception list and distributing the exception list to the plurality of sites, and recording a determination of an accept or reject result made by manual review at each relevant site;
wherein the step of determining the accept or reject result is performed at two or more of the plurality of sites, and wherein the method further comprises:
notifying the two or more of the plurality of sites when two of the sites make conflicting assertions about a common record.

2. The method according to claim 1, further including, making a positive assertion in response to the record accept result, which positive assertion asserts that the two compared records are related to the same customer; or making a negative assertion in response to the record reject result, which negative assertion asserts that the two compared records are not related to the same customer.

3. The method according to claim 2, wherein the assertions are stored in at least one of a central repository and the plurality of sites.

4. The method according to claim 1, wherein the assertions about the compared record include at least one of: the sites where the assertion was made, a person who made the assertion, and a time stamp.

5. The method according to claim 2, wherein the records that are manually reviewed are placed in one of a group specific exception list (320) and a user site specific exception list (350, 380).

6. The method according to claim 1 or 2, wherein the customers are medical patients receiving medical services at one or more of a plurality of medical facilities.

7. A computer program product comprising instructions for causing a processor system to perform the method according to any of the preceding Claims.

8. A system for matching customer records between a plurality of sites, the system comprising:
a processor configured to:
assign a unique record number to a customer record;
retrieve demographic information for a customer record in a system under consideration, to match said demographic information against demographic information in a collection of records in other systems to find records that belong to the same customer;
compare the customer record demographic information with at least one other record demographic information in the collection of records in at least one other system to derive a likelihood ratio for each compared record;
compare each likelihood ratio to a defined accept threshold and to a defined reject threshold, wherein the likelihood ratio comparison comprises one of:
- rejecting the compared customer records if the likelihood ratio falls below a reject threshold ratio,
- accepting the compared customer records if the likelihood ratio falls above an accept threshold ratio, and
- identifying the customer records for a manual review if the likelihood ratio falls between the accept threshold and the reject threshold; the processor further configured to:
attach an assertion for the compared customer records based on at least one likelihood ratio comparison; and
place the customer records to be manually reviewed on an exception list and distribute the exception list to the plurality of sites, and record a determination of an accept or reject result made by manual review at each relevant site;
wherein the step of determining the accept or reject result is performed at two or more of the plurality of sites, and wherein the processor is further configured to:
notify the two or more of the plurality of sites when two of the sites make conflicting assertions about a common record.

## Patentansprüche

1. Computerimplementiertes Verfahren zum Abgleichen von Kundenaufzeichnungen zwischen einer Vielzahl von Standorten, das Folgendes umfasst:
Zuweisen einer eindeutigen Aufzeichnungsnummer zu einer Kundenaufzeichnung (410);
Abrufen demographischer Informationen für eine Kundenaufzeichnung in einem System unter Betrachtung, um die demographischen Informationen mit demographischen Informationen in einer Sammlung von Aufzeichnungen in anderen Systemen abzugleichen, um Aufzeichnungen zu finden, die demselben Kunden (420) gehören;
Vergleichen der demographischen Informationen der Kundenaufzeichnungen mit mindestens einer anderen Aufzeichnung demographischer Informationen in der Sammlung von Aufzeichnungen in mindestens einem anderen System, um ein Wahrscheinlichkeitsverhältnis für jede verglichene Aufzeichnung (430) abzuleiten;
Vergleichen jedes Wahrscheinlichkeitsverhältnisses mit einem definierten Annahmeschwellenwert und mit einem definierten Ablehnungsschwellenwert (440), wobei das Vergleichen des Wahrscheinlichkeitsverhältnisses eines der Folgenden umfasst:
- Ablehnen der verglichenen Kundenaufzeichnungen, falls das Wahrscheinlichkeitsverhältnis unter ein Ablehnungswellenverhältnis (460) fällt,
- Akzeptieren der verglichenen Kundenaufzeichnungen, falls das Wahrscheinlichkeitsverhältnis über ein Annahmeschwellenwertverhältnis (470) fällt, und
- Identifizieren der Benutzeraufzeichnungen für eine manuelle Durchsicht, falls das Wahrscheinlichkeitsverhältnis zwischen den Annahmeschwellenwert und den Ablehnungsschwellenwert (480) fällt; wobei das Verfahren weiter Folgendes umfasst:
Anhängen einer Aussage für die verglichenen Kundenaufzeichnungen basierend auf mindestens einem Wahrscheinlichkeitsverhältnisvergleich (450); und
Platzieren der Kundenaufzeichnungen, die manuell durchzusehen sind, auf einer Ausnahmeliste, und Verteilen der Ausnahmeliste zu der Vielzahl von Standorten, und
Aufzeichnen einer Bestimmung eines Annahme- oder Ablehnungsresultats, das durch manuelle Durchsicht erfolgt, an jedem relevanten Standort;
wobei der Schritt des Bestimmens des Annahme- oder Ablehnungsresultats an zwei oder mehreren der Vielzahl von Standorten ausgeführt wird, und wobei das Verfahren weiter Folgendes umfasst:
Bekanntgeben der zwei oder mehreren der Vielzahl von Standorten, wenn zwei der Standorte gegensätzliche Aussagen über eine gemeinsame Aufzeichnung treffen.

2. Verfahren nach Anspruch 1, das weiter das Treffen einer positiven Aussage als Antwort auf das Aufzeichnungsannahmeresultat beinhaltet, wobei die positive Aussage aussagt, dass die zwei verglichenen Aufzeichnungen mit demselben Kunden zusammenhängen; oder Treffen einer negativen Aussage als Antwort auf das Aufzeichnungsablehnungsresultat, wobei die negative Aussage aussagt, dass die zwei verglichenen Aufzeichnungen nicht mit demselben Kunden zusammenhängen.

3. Verfahren nach Anspruch 2, wobei die Aussagen in mindestens einem zentralen Verwahrungsort und der Vielzahl von Standorten gespeichert werden.

4. Verfahren nach Anspruch 1, wobei die Aussagen über die verglichenen Aufzeichnung mindestens eines der Folgenden beinhaltet: die Standorte, wo die Aussage getroffen wurde, eine Person, die die Aussage getroffen hat, und einen Zeitstempel.

5. Verfahren nach Anspruch 2, wobei die Aufzeichnungen, die manuell durchgesehen werden, in eine einer Gruppe spezifischer Ausnahmeliste (320) und einer benutzerstandortspezifischen Ausnahmeliste (350, 380) platziert werden.

6. Verfahren nach Anspruch 1 oder 2, wobei die Kunden medizinische Patienten sind, die medizinische Dienstleistungen an einer oder mehreren einer Vielzahl medizinischer Einrichtungen erhalten.

7. Computerprogrammprodukt, das Anweisungen umfasst, um ein Prozessorsystem zu veranlassen, das Verfahren nach einem der vorstehenden Ansprüche auszuführen.

8. System zum Abgleichen von Kundenaufzeichnungen zwischen einer Vielzahl von Standorten, wobei das System Folgendes umfasst:
einen Prozessor, der konfiguriert ist, um:
eine eindeutige Aufzeichnungsnummer zu einer Kundenaufzeichnung zuzuweisen;
demographische Informationen für eine Kundenaufzeichnung in einem betrachteten System abzurufen, um die demographischen Informationen mit demographischen Informationen in einer Sammlung von Aufzeichnungen in anderen Systemen abzugleichen, um Aufzeichnungen zu finden, die demselben Kunden gehören;
Vergleichen der demographischen Informationen der Kundenaufzeichnung mit mindestens einer anderen Aufzeichnung demographischer Informationen in der Sammlung von Aufzeichnungen in mindestens einem anderen System, um ein Wahrscheinlichkeitsverhältnis für jede verglichene Aufzeichnung (430) abzuleiten;
Vergleichen jedes Wahrscheinlichkeitsverhältnisses mit einem definierten Annahmeschwellenwert und mit einem definierten Ablehnungsschwellenwert, wobei das Vergleichen des Wahrscheinlichkeitsverhältnisses eines der Folgenden umfasst:
- Ablehnen der verglichenen Kundenaufzeichnungen, falls das Wahrscheinlichkeitsverhältnis unter ein Ablehnungswellenverhältnis fällt,
- Akzeptieren der verglichenen Kundenaufzeichnungen, falls das Wahrscheinlichkeitsverhältnis über ein Annahmeschwellenwertverhältnis fällt, und
- Identifizieren der Benutzeraufzeichnungen für eine manuelle Durchsicht, falls das Wahrscheinlichkeitsverhältnis zwischen den Annahmeschwellenwert und den Ablehnungsschwellenwert fällt; wobei der Prozessor weiter konfiguriert ist, um:
eine Aussage für die verglichenen Kundenaufzeichnungen basierend auf mindestens einem Vergleichswahrscheinlichkeitsverhältnis anzuhängen; und
Platzieren der Kundenaufzeichnungen, die manuell durchzusehen sind, auf einer Ausnahmeliste, und Verteilen der Ausnahmeliste zu der Vielzahl von Standorten, und Aufzeichnen einer Bestimmung eines Annahme- oder Ablehnungsresultats, das durch manuelle Durchsicht erfolgt, an jedem relevanten Standort;
wobei der Schritt des Bestimmens des Annahme- oder Ablehnungsresultats an zwei oder mehreren der Vielzahl von Standorten ausgeführt wird, und wobei das Verfahren weiter konfiguriert ist, um:
die zwei oder mehreren der Vielzahl von Standorten zu verständigen, wenn zwei der Standorte gegensätzliche Aussagen über eine gemeinsame Aufzeichnung treffen.

## Revendications

1. Procédé mis en œuvre par ordinateur pour rapprocher des fichiers clients entre une pluralité de sites, comprenant :
l'attribution d'un numéro de fichier unique à un fichier client (410) ;
la récupération d'informations démographiques pour un fichier client dans un système considéré, pour associer lesdites informations démographiques et les informations démographiques dans un ensemble de fichiers dans d'autres systèmes pour trouver les fichiers qui appartiennent au même client (420) ;
la comparaison des informations démographiques du fichier client au moins aux informations démographiques d'un autre fichier dans l'ensemble de fichiers dans au moins un autre système pour déduire un rapport de probabilité pour chaque fichier comparé (430) ;
la comparaison de chaque rapport de probabilité à un seuil d'acceptation défini et à un seuil de refus défini (440), dans lequel la comparaison des rapports de probabilité comprend l'un de :
- refus des fichiers clients comparés si le rapport de probabilité se situe en dessous d'un rapport de seuils de refus (460),
- acceptation des fichiers clients comparés si le rapport de probabilité se situe au-dessus d'un rapport de seuils d'acceptation (470), et
- identification des fichiers clients en vue d'un passage en revue manuel si le rapport de probabilité se situe entre le seuil d'acceptation et le seuil de refus (480) ; le procédé comprenant en outre :
le fait de joindre une affirmation pour les fichiers clients comparés sur la base d'au moins une comparaison des rapports de probabilité (450) ; et
le fait de placer les fichiers clients devant faire l'objet d'un passage en revue manuel sur une liste d'exceptions et la distribution de la liste d'exceptions à la pluralité de sites, et l'enregistrement d'une détermination d'un résultat d'acceptation ou de refus effectuée par passage en revue manuel au niveau de chaque site concerné ;
dans lequel l'étape de détermination du résultat d'acceptation ou de refus est effectuée au niveau de deux ou plus de la pluralité de sites, et dans lequel le procédé comprend en outre :
la notification des deux ou plus de la pluralité de sites lorsque deux des sites font des affirmations conflictuelles concernant un fichier commun.

2. Procédé selon la revendication 1, incluant en outre, le fait d'avancer une affirmation positive en réponse au résultat d'acceptation du fichier, laquelle l'affirmation positive affirme que les deux fichiers comparés sont liés au même client ; ou le fait de faire une affirmation négative en réponse au résultat de refus du fichier, laquelle affirmation négative affirme que les deux fichiers comparés ne sont pas liés aux même client.

3. Procédé selon la revendication 2, dans lequel les affirmations sont stockées dans au moins l'un d'un dépôt central et de la pluralité de sites.

4. Procédé selon la revendication 1, dans lequel les affirmations concernant le fichier comparé incluent au moins l'un de : les sites où l'affirmation a été faite, une personne qui a fait l'affirmation, et une estampille temporelle.

5. Procédé selon la revendication 2, dans lequel les fichiers qui sont passés en revue manuellement sont placés dans l'une d'une liste d'exceptions spécifique à un groupe (320) et d'une liste d'exceptions spécifique à un site utilisateur (350, 380).

6. Procédé selon la revendication 1 ou 2, dans lequel les clients sont des patients médicaux recevant des services médicaux au niveau d'une ou plusieurs d'une pluralité d'installations médicales.

7. Produit de programme informatique comprenant des instructions pour amener un système de processeur à exécuter le procédé selon l'une quelconque des revendications précédentes.

8. Système pour associer des fichiers clients entre une pluralité de sites, le système comprenant :
un processeur configuré pour :
attribuer un numéro de fichier unique à un fichier client ;
récupérer des informations démographiques pour un fichier client dans un système considéré, pour associer lesdites informations démographiques et les informations démographiques dans un ensemble de fichiers dans d'autres systèmes pour trouver les fichiers qui appartiennent au même client ;
comparer les informations démographiques du fichier client au moins aux informations démographiques d'un autre fichier dans l'ensemble de fichiers dans au moins un autre système pour déduire un rapport de probabilité pour chaque fichier comparé ;
comparer chaque rapport de probabilité à un seuil d'acceptation défini et à un seuil de refus défini, dans lequel la comparaison des rapports de probabilité comprend l'un de :
- refus des fichiers clients comparés si le rapport de probabilité se situe en dessous d'un rapport de seuils de refus,
- acceptation des fichiers clients comparés si le rapport de probabilité se situe au-dessus d'un rapport de seuils d'acceptation, et
- identification des fichiers clients en vue d'un passage en revue manuel si le rapport de probabilité se situe entre le seuil d'acceptation et le seuil de refus ; le processeur en outre configuré pour :
joindre une affirmation pour les fichiers clients comparés sur la base d'au moins une comparaison des rapports de probabilité ; et
placer les fichiers clients devant faire l'objet d'un passage en revue manuel sur une liste d'exceptions et distribuer la liste d'exceptions à la pluralité de sites, et enregistrer une détermination d'un résultat d'acceptation ou de refus effectué par passage en revue manuel au niveau de chaque site concerné ;
dans lequel l'étape de détermination du résultat d'acceptation ou de refus est effectuée au niveau de deux ou plus de la pluralité de sites, et dans lequel le processeur est en outre configuré pour :
notifier les deux ou plus de la pluralité de sites lorsque deux des sites font des affirmations conflictuelles concernant un fichier commun.
